# EUROPÄISCHE PATENTSCHRIFT

(11) **EP 1 153 917 B1**
(45) Veröffentlichungstag und Bekanntmachung des Hinweises auf die Patenterteilung: **13.04.2005**
(21) Anmeldenummer: 01103708.2
(22) Anmeldetag: 15.02.2001
(51) Int. Cl.: C07D 207/335, C07D 307/72, C07D 333/20, C07D 333/22, C07D 333/28, C07D 333/32, C07D 333/42, A61K 7/13

(54) **p-Aminophenol-Derivate und diese enthaltende Färbemittel**
p-Aminophenol derivatives and colorants containing them
Dérivés de p-aminophénol et colorants les contenant

(30) Priorität: 10.05.2000 DE 10022829
(43) Veröffentlichungstag der Anmeldung: 14.11.2001
(73) Patentinhaber: Wella Aktiengesellschaft, 64295 Darmstadt (DE)
(72) Erfinder: Chassot, Laurent, 1724 Praroman (CH); Braun, Hans-Jürgen, 3182 Überstorf (CH)

(56) Entgegenhaltungen:
- WO-A-01/51019
- DE-A- 19 607 751

## Beschreibung

Die Erfindung betrifft neue, in 2-Stellung substituierte p-Aminophenol-Derivate sowie diese Verbindungen enthaltende Mittel zum Färben von Keratinfasern, insbesondere menschlichen Haaren.

Auf dem Gebiet der Färbung von Keratinfasern, insbesondere der Haarfärbung, haben Oxidationsfarbstoffe eine wesentliche Bedeutung erlangt. Die Färbung entsteht hierbei durch Reaktion bestimmter Entwicklersubstanzen mit bestimmten Kupplersubstanzen in Gegenwart eines geeigneten Oxidationsmittels. Als Entwicklersubstanzen werden hierbei insbesondere 2,5-Diaminotoluol, 2,5-Diaminophenylethylalkohol, p-Aminophenol und 1,4-Diaminobenzol eingesetzt, während als Kupplersubstanzen beispielsweise Resorcin, 4-Chlorresorcin, 1-Naphthol, 3-Aminophenol, 5-Amino-2-methylphenol und Derivate des m-Phenylendiamins zu nennen sind. An Oxidationsfarbstoffe, die zur Färbung menschlicher Haare verwendet werden, werden neben der Färbung in der gewünschten Intensität zahlreiche zusätzliche Anforderungen gestellt. So müssen die Farbstoffe in toxikologischer und dermatologischer Hinsicht unbedenklich sein und die erzielten Haarfärbungen eine gute Lichtechtheit, Dauerwellechtheit, Säureechtheit und Reibeechtheit aufweisen. Auf jeden Fall aber müssen solche Färbungen ohne Einwirkung von Licht, Reibung und chemischen Mitteln über einen Zeitraum von mindestens 4 bis 6 Wochen stabil bleiben. Außerdem ist es erforderlich, daß durch Kombination geeigneter Entwicklersubstanzen und Kupplersubstanzen eine breite Palette verschiedener Farbnuancen erzeugt werden kann. Zur Erzielung nartürlicher und besonders modischer Nuancen im Rotbereich wird vor allem p-Aminophenol, allein oder im Gemisch mit anderen Entwicklersubstanzen, in Kombination mit geeigneten Kupplersubstanzen eingesetzt. Es wurde bereits versucht, die Eigenschaften von p-Aminophenolen durch die Einführung von Substituenten zu verbessern. In diesem Zusammenhang sei auf die DE-OS 196 07 751 verwiesen, in der unter anderem auch Färbemittel beschrieben, welche als Entwicklersubstanzen spezielle, in 2-Stellung substituierte p-Aminophenol enthalten.

Mit den derzeit bekannten Färbemitteln ist es jedoch nicht möglich, die an ein Färbemittel gestellten Anforderungen in allen Punkten zu erfüllen. Es bestand daher weiterhin ein Bedürfnis nach neuen Entwicklersubstanzen, welche die vorgenannten Anforderung in besonderem Masse erfüllen.

Hierzu wurde nun überraschenderweise gefunden, daß neue p-Aminophenol-Derivate gemäß der allgemeinen Formel (I) die an Entwicklersubstanzen gestellten Anforderungen in besonders hohem Masse erfüllen. So werden bei Verwendung dieser Entwicklersubstanzen mit den meisten bekannten Kupplersubstanzen farbstarke Farbnuancen erhalten, die außerordentlich lichtecht und waschecht sind.

Gegenstand der vorliegende Erfindung sind daher p-Aminophenol-Derivate der allgemeinen Formel (I) oder deren physiologisch verträgliche, wasserlösliche Salze, 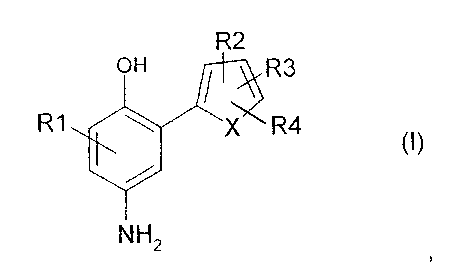 worin
**X** gleich Sauerstoff, Schwefel, oder NR5 ist;
**R1** gleich Wasserstoff, einem Halogenatom, einer C₁-C₄-Alkylgruppe, einer C₁-C₄-Hydroxyalkylgruppe oder einer C₁-C₄-Alkoxygruppe ist;
**R2** und **R4** unabhänging voneinander gleich Wasserstoff, einer Hydroxygruppe, einem Halogenatom, einer Cyanogruppe, einer C₁-C₄-Alkoxygruppe, einer C₁-C₆-Alkylgruppe, einer C₁-C₄-Alkylthioethergruppe, einer Mercaptogruppe, einer Nitrogruppe, einer Aminogruppe, einer C₁-C₆-Alkylaminogruppe, einer (C₁-C₆)-Dialkylaminogruppe, einer -C(O)H-Gruppe, einer -C(O)CH₃-Gruppe, einer-C(O)CF₃-Gruppe, einer -Si(CH₃)₃-Gruppe, einer C₁-C₄-Hydroxyalkylgruppe, einer C₃-C₄-Dihydroxyalkylgruppe, einer -CH=CHR6-Gruppe, einer -(CH₂)ₚ-CO₂R7-Gruppe oder einer -(CH₂)ₚ-R8-Gruppe (mit p=1,2,3 oder 4), einer -C(R9)=NR10-Gruppe oder einer C(R11)H-NR12R13-Gruppe sind;
**R3** gleich Wasserstoff, einem Halogenatom, einer C₁-C₆ Alkylgruppe oder einer -C(O)H-Gruppe ist;
**R5** gleich Wasserstoff, einer C₁-C₆-Alkylgruppe, einer C₁-C₄-Hydroxyalkylgruppe, einer Phenylgruppe oder einer Acetylgruppe ist;
**R6** gleich Wasserstoff, einer Hydroxygruppe, einer Nitrogruppe, einer Aminogruppe, einer -CO₂R7-Gruppe oder einer -C(O)CH₃-Gruppe ist;
**R7, R9 und R11** unabhänging voneinander gleich Wasserstoff oder einer C₁-C₄-Alkylgruppe sind;
**R8** gleich einer Aminogruppe oder einer Nitrilgruppe ist;
**R10, R12 und R13** unabhängig voneinander gleich Wasserstoff, einer Hydroxygruppe, einer C₁-C₄-Alkylgruppe, einer C₁-C₄-Hydroxyalkylgruppe, einer C₃-C₄-Dihydroxyalkylgruppe oder einem Rest der Formel (II) sind 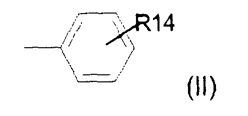 und
**R14** gleich Wasserstoff, einer Aminogruppe oder einer Hydroxygruppe ist, unter der Bedingung, dass mindestens einer der Reste **R1** bis **R4** von Wasserstoff verschieden ist, wenn **X** gleich Schwefel ist.

Als Verbindungen der Formel (I) können insbesondere genannt werden: 4-Amino-2-(2-furyl)-phenol; 4-Amino-2-(pyrrol-2-yl)-phenol; 4-Amino-2-(1-methyl-1H-pyrrol-2-yl)-phenol; 4-Amino-3-chlor-2-(2-thienyl)-phenol; 4-Amino-3-methyl-2-(2-thienyl)-phenol; 4-Amino-5-chlor-2-(2-thienyl)-phenol; 4-Amino-5-methyl-2-(2-thienyl)-phenol; 4-Amino-6-chlor-2-(2-thienyl)-phenol; 4-Amino-6-methyl-2-(2-thienyl)-phenol; 4-Amino-2-(3-acetyl-2-thienyl)-phenol; 4-Amino-2-(3-chlor-2-thienyl)-phenol; 4-Amino-2-(3-formyl-2-thienyl)-phenol; 4-Amino-2-(3-methyl-2-thienyl)-phenol; 4-Amino-2-(3-nitro-2-thienyl)-phenol; 4-Amino-2-(4-acetyl-2-thienyl)-phenol; 4-Amino-2-(4-chlor-2-thienyl)-phenol; 4-Amino-2-(4-formyl-2-thienyl)-phenol; 4-Amino-2-(4-methyl-2-thienyl)-phenol; 4-Amino-2-(4-nitro-2-thienyl)-phenol; 4-Amino-2-(5-acetyl-2-thienyl)-phenol; 4-Amino-2-(5-chlor-2-thienyl)-phenol; 4-Amino-2-(5-formyl-2-thienyl)-phenol; 4-Amino-2-(5-methyl-2-thienyl)-phenol; 4-Amino-2-(5-nitro-2-thienyl)-phenol sowie deren physiologisch verträgliche Salze.

Bevorzugt sind hierbei Verbindungen der Formel (I), in denen (i) **R1** Wasserstoff bedeutet und/oder (ii) mindestens einer der Reste **R2, R3** und **R4** Wasserstoff oder eine Methylgruppe bedeuten und/oder (iii) **X** Schwefel oder Sauerstoff bedeutet, unter der Bedingung, dass mindestens einer der Reste **R1** bis **R4** von Wasserstoff verschieden ist, wenn **X** gleich Schwefel ist.

Besonders bevorzugte p-Aminophenol-Derivate der Formel (I) sind hierbei 4-Amino-2-(3-methyl-2-thienyl)-phenol und 4-Amino-2-(5-methyl-2-thienyl)-phenol sowie deren physiologisch verträgliche Salze.

Die Verbindungen der Formel (I) können sowohl als freie Basen als auch in Form ihrer physiologisch verträglichen Salze mit anorganischen oder organischen Säuren, wie zum Beispiel Salzsäure, Schwefelsäure, Phosphorsäure, Essigsäure, Propionsäure, Milchsäure oder Zitronensäure, eingesetzt werden.

Die Herstellung der erfindungsgemäßen Aminophenol-Derivate der Formel (I) kann unter Verwendung von literaturbekannten Syntheseverfahren erfolgen, beispielsweise durch eine Palladium(0) katalysierte Kupplung eines substituierten Benzols der Formel (II) mit einer Heteroarylverbindung der Formel (III) 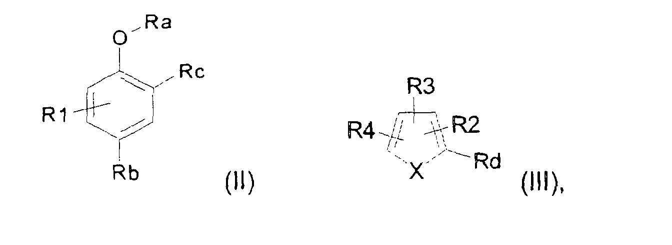 und anschließende Abspaltung der Schutzgruppe, gegebenenfalls gefolgt von einer Reduktion der Nitrogruppe; wobei die Restgruppen in den Formeln (II) und (III) die folgende Bedeutung haben: **Ra** steht für eine Schutzgruppe -wie sie zum Beispiel in dem Kapitel "Protective Groups" in Organic Synthesis, Kapitel 7, Wiley Interscience, 1991 beschrieben wird- und **Rb** für eine NHRa-Gruppe oder eine Nitrogruppe; einer der beiden Reste **Rc** und **Rd** steht für eine Halogengruppe während der andere Rest eine B(OH)-Gruppe darstellt, und **X, R1, R2, R3** und **R4** haben die in Formel (I) gennante Bedeutung.

Die Verbindungen sind insbesondere als Entwicklersubstanzen in Oxidationsfärbemittel verwendbar und ermöglichen eine breite Palette verschiedener Farbnuancen, welche sich von blonden über braune, purpurne, violette bis hin zu blauen und schwarzen Farbtönen erstrecken.

Ein weiterer Gegenstand der vorliegenden Erfindung sind daher Mittel zum oxidativen Färben von Keratinfasern, wie zum Beispiel Haaren, Pelzen, Federn oder Wolle, insbesondere menschlichen Haaren, auf der Basis einer Entwicklersubstanz-Kupplersubstanz-Kombination, welche als Entwicklersubstanz mindestens ein p-Aminophenol-Derivat der Formel (I) enthalten.

Das Aminophenol-Derivat der Formel (I) ist in dem erfindungsgemäßen Färbemittel in einer Menge von etwa 0,005 bis 20 Gewichtsprozent enthalten, wobei eine Menge von etwa 0,01 bis 5,0 Gewichtsprozent und insbesondere 0,1 bis 2,5 Gewichtsprozent bevorzugt ist.

Als Kupplersubstanzen kommen vorzugsweise 2,6-Diamino-pyridin, 2-Amino-4-[(2-hydroxyethyl)amino]-anisol, 2,4-Diamino-1-fluor-5-methylbenzol, 2,4-Diamino-1-methoxy-5-methyl-benzol, 2,4-Diamino-1-ethoxy-5-methyl-benzol, 2,4-Diamino-1-(2-hydroxyethoxy)-5-methyl-benzol, 2,4-Di[(2-hydroxyethyl)amino]-1,5-dimethoxy-benzol, 2,3-Diamino-6-methoxy-pyridin, 3-Amino-6-methoxy-2-(methylamino)-pyridin, 2,6-Diamino-3,5-dimethoxy-pyridin, 3,5-Diamino-2,6-dimethoxy-pyridin, 1,3-Diamino-benzol, 2,4-Diamino-1-(2-hydroxyethoxy)-benzol, 2,4-Diamino-1,5-di(2-hydroxyethoxy)-benzol, 1-(2-Aminoethoxy)-2,4-diamino-benzol, 2-Amino-1-(2-hydroxyethoxy)-4-methylamino-benzol, 2,4-Diaminophenoxy-essigsäure, 3-[Di(2-hydroxyethyl)amino]-anilin, 4-Amino-2-di[(2-hydroxyethyl)amino]-1-ethoxy-benzol, 5-Methyl-2-(1-methylethyl)-phenol, 3-[(2-Hydroxyethyl)amino]-anilin, 3-[(2-Aminoethyl)-amino]-anilin, 1,3-Di(2,4-diaminophenoxy)-propan, Di(2,4-diaminophenoxy)-methan, 1,3-Diamino-2,4-dimethoxy-benzol, 2,6-Bis(2-hydroxyethyl)amino-toluol, 4-Hydroxyindol, 3-Dimethylamino-phenol, 3-Diethylamino-phenol, 5-Amino-2-methyl-phenol, 5-Amino-4-fluor-2-methylphenol, 5-Amino-4-methoxy-2-methyl-phenol, 5-Amino-4-ethoxy-2-methylphenol, 3-Amino-2,4-dichlor-phenol, 5-Amino-2,4-dichlor-phenol, 3-Amino-2-methyl-phenol, 3-Amino-2-chlor-6-methyl-phenol, 3-Amino-phenol, 2-[(3-Hydroxyphenyl)amino]-acetamid, 5-[(2-Hydroxyethyl)amino]-2-methyl-phenol, 3-[(2-Hydroxyethyl)amino]-phenol, 3-[(2-Methoxyethyl)-amino]-phenol, 5-Amino-2-ethyl-phenol, 2-(4-Amino-2-hydroxyphenoxy)-ethanol, 5-[(3-Hydroxypropyl)amino]-2-methyl-phenol, 3-[(2,3-Dihydroxypropyl)amino]-2-methyl-phenol, 3-[(2-Hydroxyethyl)amino]-2-methylphenol, 2-Amino-3-hydroxy-pyridin, 5-Amino-4-chlor-2-methyl-phenol, 1-Naphthol, 1,5-Dihydroxy-naphthalin, 1,7-Dihydroxy-naphthalin, 2,3-Dihydroxy-naphthalin, 2,7-Dihydroxy-naphthalin, 2-Methyl-1-naphtholacetat, 1,3-Dihydroxy-benzol, 1-Chlor-2,4-dihydroxy-benzol, 2-Chlor-1,3-dihydroxy-benzol, 1,2-Dichlor-3,5-dihydroxy-4-methyl-benzol, 1,5-Dichlor-2,4-dihydroxy-benzol, 1,3-Dihydroxy-2-methyl-benzol, 3,4-Methylendioxy-phenol, 3,4-Methylendioxy-anilin, 5-[(2-Hydroxyethyl)amino]-1,3-benzodioxol, 6-Brom-1-hydroxy-3,4-methylendioxy-benzol, 3,4-Diamino-benzoesäure, 3,4-Dihydro-6-hydroxy-1,4(2H)-benzoxazin, 6-Amino-3,4-dihydro-1,4(2H)-benzoxazin, 3-Methyl-1-phenyl-5-pyrazolon, 5,6-Dihydroxy-indol, 5,6-Dihydroxy-indolin, 5-Hydroxy-indol, 6-Hydroxy-indol, 7-Hydroxy-indol und 2,3-Indolindion in Betracht.

Obwohl die vorteilhaften Eigenschaften der hier beschriebenen Aminophenol-Derivate der Formel (I) es nahelegen, diese als alleinige Entwicklersubstanz zu verwenden, ist es selbstverständlich auch möglich, die Aminophenol-Derivate der Formel (I) gemeinsam mit bekannten Entwicklersubstanzen, wie zum Beispiel 1,4-Diaminobenzol, 2,5-Diaminotoluol, 2,5-Diaminophenylethylalkohol, N,N-Bis-(2'-hydroxyethyl)-1,4-diaminobenzol, 4-Aminophenol und dessen Derivaten, wie zum Beispiel 4-Amino-3-methylphenol, 4,5-Diamino-1-(2-hydroxyethyl)-pyrazol oder Tetraaminopyrimidinen, einzusetzen.

Die Kupplersubstanzen und Entwicklersubstanzen können in dem erfindungsgemäßen Färbemittel jeweils einzeln oder im Gemisch miteinander enthalten sein, wobei die Gesamtmenge an Kupplersubstanzen und Entwicklersubstanzen in dem erfindungsgemäßen Färbemittel (bezogen auf die Gesamtmenge des Färbemittels) jeweils etwa 0,005 bis 20 Gewichtsprozent, vorzugsweise etwa 0,01 bis 5,0 Gewichtsprozent und insbesondere 0,1 bis 2,5 Gewichtsprozent, beträgt.

Die Gesamtmenge der in dem hier beschriebenen Färbemittel enthaltenen Entwicklersubstanz-Kupplersubstanz-Kombination beträgt vorzugsweise etwa 0,01 bis 20 Gewichtsprozent, wobei eine Menge von etwa 0,02 bis 10 Gewichtsprozent und insbesondere 0,2 bis 6,0 Gewichtsprozent besonders bevorzugt ist. Die Entwicklersubstanzen und Kupplersubstanzen werden im allgemeinen in etwa äquimolaren Mengen eingesetzt; es ist jedoch nicht nachteilig, wenn die Entwicklersubstanzen diesbezüglich in einem gewissen Überschuß oder Unterschuß vorhanden sind.

Weiterhin kann das erfindungsgemäße Färbemittel zusätzlich andere Farbstoffe, beispielsweise 6-Amino-2-methylphenol und 2-Amino-5-methylphenol, und übliche direktziehende Farbstoffe, zum Beispiel Triphenylmethanfarbstoffe wie 4-[(4'-aminophenyl)-(4'imino-2",5"cyclohexadien-1"-yliden)-methyl]-2-methylaminobenzol-monohydrochlorid (C.I. 42 510) und 4-[(4'amino-3'-methyl-phenyl)-(4"-imino-3"-methyl-2",5"cyclohexadien-1 "-yliden)-methyl]-2-methyl-aminobenzolmonohydrochlorid (C.I. 42 520), aromatische Nitrofarbstoffe wie 4-(2'-hydroxyethyl)amino-nitrotoluol, 2-Amino-4,6-dinitrophenol, 2-Amino-5-(2'-hydroxyethyl)amino-nitrobenzol, 2-Chlor-6-(ethylamino)-4-nitrophenol, 4-Chlor-N-(2-hydroxyethyl)-2-nitroanilin, 5-Chlor-2-hydroxy-4-nitroanilin, 2-Amino-4-chlor-6-nitrophenol und 1-[(2'-Ureidoethyl)amino-4-nitrobenzol, Azofarbstoffe wie 6-[(4'-Aminophenyl)azo]-5hydroxynaphthalin-1-sulfonsäure-Natriumsatz (C.I. 14 805), Dispersionsfarbstoffe wie beispielsweise 1,4-Diaminoanthrachinon und 1,4,5,8-Tetraaminoantrachinon, oder kationische Farbstoffe enthalten.
Die Färbemittel können diese Farbstoffe in einer Menge von etwa 0,1 bis 4,0 Gewichtsprozent enthalten.

Selbstverständlich können die Kupplersubstanzen und Entwicklersubstanzen sowie die anderen Farbstoffe, sofern es Basen sind, auch in Form ihrer physiologisch verträglichen Salze mit organischen oder anorganischen Säuren, beispielsweise Salzsäure oder Schwefelsäure, beziehungsweise -sofern sie aromatische OH-Gruppen besitzen- in Form ihrer Salze mit Basen, beispielsweise als Alkaliphenolate, eingesetzt werden.

Darüber hinaus können in den Färbemitteln, falls diese zur Färbung von Haaren verwendet werden sollen, noch weitere übliche kosmetische Zusätze, beispielsweise Antioxidantien wie Ascorbinsäure, Thioglykolsäure oder Natriumsulfit, sowie Parfümöle, Komplexbildner, Netzmittel, Emulgatoren, Verdicker und Pflegestoffe enthalten sein.

Die Zubereitungsform des erfindungsgemäßen Färbemittels kann beispielsweise eine Lösung, insbesondere eine wäßrige oder wäßrigalkoholische Lösung sein. Die besonders bevorzugten Zubereitungsformen sind jedoch eine Creme, ein Gel oder eine Emulsion. Ihre Zusammensetzung stellt eine Mischung der Farbstoffkomponenten mit den für solche Zubereitungen üblichen Zusätzen dar.

Ubliche Zusätze in Lösungen, Cremes, Emulsionen oder Gelen sind zum Beispiel Lösungsmittel wie Wasser, niedere aliphatische Alkohole, beispielsweise Ethanol, Propanol oder Isopropanol, Glycerin oder Glykole wie 1,2-Propylenglykol, weiterhin Netzmittel oder Emulgatoren aus den Klassen der anionischen, kationischen, amphoteren oder nichtionogenen oberflächenaktiven Substanzen wie zum Beispiel Fettalkoholsulfate, oxethylierte Fettalkoholsulfate, Alkylsulfonate, Alkylbenzolsulfonate, Alkyltrimethylammoniumsalze, Alkylbetaine, oxethylierte Fettalkohole, oxethylierte Nonylphenole, Fettsäurealkanolamide und oxethylierte Fettsäureester ferner Verdicker wie hohere Fettalkohole, Stärke, Cellulosederivate, Petrolatum, Paraffinöl und Fettsäuren, sowie außerdem Pflegestoffe wie kationische Harze, Lanolinderivate, Cholesterin, Pantothensäure und Betain. Die erwähnten Bestandteile werden in den für solche Zwecke üblichen Mengen verwendet, zum Beispiel die Netzmittel und Emulgatoren in Konzentrationen von etwa 0,5 bis 30 Gewichtsprozent, die Verdicker in einer Menge von etwa 0,1 bis 25 Gewichtsprozent und die Pflegestoffe in einer Konzentration von etwa 0,1 bis 5,0 Gewichtsprozent.

Je nach Zusammensetzung kann das erfindungsgemäße Färbemittel schwach sauer, neutral oder alkalisch reagieren. Insbesondere weist es einen pH-Wert von etwa 6,5 bis 11,5 auf. Die basische Einstellung erfolgt hierbei vorzugsweise mit Ammoniak, es können aber auch organische Amine, zum Beispiel Monoethanolamin und Triethanolamin, oder anorganische Basen wie Natriumhydroxid und Kaliumhydroxid Verwendung finden. Für eine pH-Einstellung im sauren Bereich können anorganische oder organische Säuren, beispielsweise Phosphorsäure, Essigsäure, Zitronensäure oder Weinsäure, verwendet werden.

Für die Anwendung zur oxidativen Färbung von Haaren vermischt man das vorstehend beschriebene Färbemittel unmittelbar vor dem Gebrauch mit einem Oxidationsmittel und trägt eine für die Haarfärbebehandlung ausreichende Menge, je nach Haarfülle, im allgemeinen etwa 60 bis 200 Gramm, dieses Gemisches auf das Haar auf.

Als Oxidationsmittel zur Entwicklung der Haarfärbung kommen hauptsächlich Wasserstoffperoxid oder dessen Additionsverbindungen an Harnstoff, Melamin, Natriumborat oder Natriumcarbonat in Form einer 3-bis 12prozentigen, vorzugsweise 6prozentigen, wässrigen Lösung, aber auch Luftsauerstoff in Betracht. Wird eine 6prozentige Wasserstoffperoxid-Lösung als Oxidationsmittel verwendet, so beträgt das Gewichtsverhältnis zwischen Haarfärbemittel und Oxidationsmittel 5:1 bis 1:2, vorzugsweise jedoch 1:1. Größere Mengen an Oxidationsmittel werden vor allem bei höheren Farbstoffkonzentrationen im Haarfärbemittel, oder wenn gleichzeitig eine stärkere Bleichung des Haares beabsichtigt ist, verwendet. Man läßt das Gemisch bei 15 bis 50 Grad Celsius etwa 10 bis 45 Minuten lang, vorzugsweise 30 Minuten lang, auf das Haar einwirken, spült sodann das Haar mit Wasser aus und trocknet es. Gegebenenfalls wird im Anschluß an diese Spülung mit einem Shampoo gewaschen und eventuell mit einer schwachen organischen Säure, wie zum Beispiel Zitronensäure oder Weinsäure, nachgespült. Anschließend wird das Haar getrocknet.

Die erfindungsgemäßen Haarfärbemittel mit einem Gehalt an Aminophenol-Derivaten der Formel (I) als Entwicklersubstanz ermöglichen Haarfärbungen mit ausgezeichneter Farbechtheit, insbesondere was die Lichtechtheit, Waschechtheit und Reibeechtheit anbetrifft. Hinsichtlich der färberischen Eigenschaften bieten die erfindungsgemäßen Haarfärbemittel je nach Art und Zusammensetzung der Farbkomponenten eine breite Palette verschiedener Farbnuancen, welche sich von blonden über braune, purpurne, violette bis hin zu blauen und schwarzen Farbtönen erstreckt. Hierbei zeichnen sich die Farbtöne durch ihre besondere Farbintensität und einen guten Farbausgleich zwischen geschädigtem und Geschädigtem Haar aus. Die sehr guten färberischen Eigenschaften der Haarfärbemittel gemäß der vorliegenden Anmeldung zeigen sich weiterhin darin, daß diese Mittel eine Anfärbung von ergrauten, chemisch nicht vorgeschädigten Haaren problemlos und mit guter Deckkraft ermöglichen.

Die nachfolgenden Beispiele sollen den Gegenstand der Erfindung näher erläutern.

### Beispiele

### Beispiel 1: Synthese von 4-Amino-2-(2-heteroaryl)-phenol Derivaten der Formel (I) (Allgemeine Synthesevorschrift)

### A. Synthese von N-(3-Brom-4-hydroxy-phenyl)-carbaminsäuretert.butylester

Zu einer Suspension von N(4-Hydroxy-phenyl)-carbaminsäuretert.butylester (10g, 47,8 mmol) in Chloroform (100 ml) tropft man innerhalb von 2 Stunden bei 0 °C eine Lösung von 9,4g N-Bromsuccinimid (52,8mmol) in 450 ml Chloroform. Die Reaktionsmischung wird anschließend weitere 15 Minuten gerührt, sodann zweimal mit Wasser (zuerst 400ml, dann 200ml) gewaschen, mit Magnesiumsulfat getrocknet, filtriert und teilweise eingeengt. Der Rückstand wird sodann unter Rühren mit Hexan versetzt, wobei sich ein Niederschlag bildet. Der Niederschlag wird abfiltriert und mit Hexan gewaschen.
Es werden 9,7 g (70 % der Theorie) N-(3-Brom-4-hydroxy-phenyl)-carbaminsäure-tert.butylester erhalten.
¹H-NMR (300 MHz, CDCl₃): δ= 7,68 (br s, 1H); 7,05 (dd, 1H); 6,93 (d, 1H); 6,37 (br s; 2H); 5,39 (s, 1H); 1,51 (s, 9H)

### B. Synthese von N-(3-Brom-4-ethoxymethoxy-phenyl)-carbaminsäuretert.butylester

Zu einer Lösung von 5 g N-(3-Brom-4-hydroxy-phenyl)-carbaminsäuretert.butylester (17,4 mmol) in 60 ml Teratohydrofuran werden bei 0°C portionenweise 0,76 g (17,4 mmol) einer Natriumhydrid-Dispersion (55% in Öl) gegeben. Das Gemisch wird anschließend 50 Minuten lang bei 0 °C gerührt und sodann mit 1,83 g Chlormethyl-ethylether (19,4 mmol) versetzt. Das Gemisch wird eine weiter Stunde lang bei 0°C gerührt und sodann auf Eis gegossen, mit Essigsäureethylester extrahiert und die organische Phase mit einer gesättigten wässrigen Kochsalz-Lösung gewaschen, über Na2SO4 getrocknet und nach Filtration eingeengt.
Der Rückstand wird an Kieselgel mit Petrolether/Essigsäureethylester (9:1) gereinigt.
Es werden 4,8 g (80 % der Theorie) N-(3-Brom-4-hydroxy-phenyl)-carbaminsäure-tert.butylester erhalten.
¹H-NMR (300 MHz, CDCl₃): δ= 7,66 (d, 1H); 7,16 (dd, 1H); 7,08 (d, 1H); 5,23 (s; 2H); 3,77 (q, 2H); 1,51 (s, 9H); 1,22 (t, 3H)

### C. Synthese von N-[4-Ethoxymethoxy-3-(4,4,5,5-tetramethyl-[1,3,2]dioxaborolan-2-yl)-phenyl]-carbaminsäure-tert.butylester

Eine Mischung aus 7,0 g (20,2 mmol) N-(3-Brom-4-ethoxymethoxyphenyl)-carbaminsäure-tert.butylester, 12,8 g (50,6 mmol) Diboronpinacolester, 2,0 g (2,9 mmol) Dichloro(1,1'-bis(diphenylphosphino)ferrocene)palladium (PdCl₂(dppf)) und 6,2 g (63,2 mmol) Kaliumacetat werden unter Argon mit 210 ml entgastem Dioxan versetzt . Das Gemisch wird anschließend 26 Stunden lang bei 80°C gerührt, sodann mit einem Gemisch aus 4,2 g (16,9 mmol) Diboronpinacolester und 0,7 g (0,95 mmol) PdCl₂(dppf) versetzt und erneut für 14 Stunden bei 80°C gerührt. Anschließend wird das Reaktionsgemisch auf Wasser gegossen, mit Essigsäureethylester extrahiert und die organische Phase mit einer gesättigten wässrigen Kochsalz-Lösung gewaschen, über Na2SO₄ getrocknet und nach der Filtration eingeengt.
Das Rohprodukt wird abschließend an desaktiviertem Kieselgel mit Hexan/Essigsäureethylester gereinigt.
Es werden 5,30 g (61 % der Theorie) N-[4-Ethoxymethoxy-3-(4,4,5,5-tetramethyl-[1,3,2]dioxaborolan-2-yl)-phenyl]- carbaminsäuretert.butylester erhalten.

### D. Synthese von 4-Amino-2-(2-heteroaryl)phenolen

0,036 g (0,1 mmol) N-[4-Ethoxymethoxy-3-(4,4,5,5-tetramethyl-[1,3,2]dioxaborolan-2-yl)-phenyl]-carbaminsäure-tert.butylester und 0,013 mol des entsprechenden Bromderivates werden unter Argon in 70 ml 1,2-Dimethoxyethan gelöst. Anschließend werden 0,5 g (0,5 mmol) Tetrakis-(triphenylphosphin)-palladium und 13 ml einer 2normalen Kaliumcarbonat-Lösung zugegeben und die Reaktionsmischung auf 80 °C erwärmt. Nach Beendigung der Reaktion wird die Reaktionsmischung in 100 ml Essigsäureethylester gegossen, die organische Phase mit verdünnter Natronlauge extrahiert und sodann mit Magnesiumsulfat getrocknet. Das Lösungsmittel wird am Rotationsverdampfer abdestilliert und der Rückstand an Kieselgel mit Petrolether/Essigsäureethylester (9:1) gereinigt. Das so erhaltene Produkt wird in 40 ml Ethanol auf 50 °C erwärmt. Anschließend werden zur Herstellung des Hydrochlorides 15 ml einer 2,9molaren ethanolische Salzsäurelösung zugetropft. Der Niederschlag wird abfiltriert, zweimal mit 10 ml Ethanol gewaschen und sodann getrocknet.

### a. 4-Amino-2-(5-methyl-thiophen-2-yl)-phenol Hydrochlorid

Verwendete Bromderivat: 2-Brom-5-methyl-thiophen
Ausbeute: 0,025 g (98 % der Theorie)
Masspektrum: MH⁺ 206 (100)

### b. 4-Amino-2-(5-nitro-thiophen-2-yl)-phenol Hydrochlorid

Verwendete Bromderivat: 2-Brom-5-nitro-thiophen
Ausbeute: 0,025 g (93 % der Theorie)
Masspektrum: MH⁺237 (85)

### c. 4-Amino-2-furan-2-yl-phenol Hydrochlorid

Verwendete Bromderivat: 2-Brom-furan
Ausbeute: 0,012 g (53 % der Theorie)
Masspektrum: M-H⁺ 176 (100)

### Beispiele 2 bis 13: Haarfärbemittel

Es werden Haarfärbelösungen der folgenden Zusammensetzung hergestellt:

| | |
|---|---|
| 1,25 mmol | Entwicklersubstanz der Formel (I) gemäss Tabelle 1 |
| 1,25 mmol | Kupplersubstanz gemäß Tabelle 1 |
| 1,0 g | Kaliumoleat (8prozentige wässrige Lösung) |
| 1,0 g | Ammoniak (22prozentige wässrige Lösung) |
| 1,0 g | Ethanol |
| 0, 3 g | Ascorbinsäure |
| ad 100,0 g | Wasser |

30 g der vorstehenden Färbelösung werden unmittelbar vor der Anwendung mit 30 g einer 6prozentigen wässrigen Wasserstoffperoxidlösung vermischt. Anschließsend wird das Gemisch auf gebleichte Haare aufgetragen. Nach einer Einwirkungszeit von 30 Minuten bei 40 °C wird das Haar mit Wasser gespült, mit einem handelsüblichen Shampoo gewaschen und getrocknet. Die resultierenden Färbungen sind in Tabelle 1 zusammengefaßt.

**Tabelle 1:**

| **Beispiel/ Farbton** | **Entwicklersubstanz der Formel (I)** | **Kupplersubstanz** |
|---|---|---|
| **3** | 4-Amino-2-(2-thienyl)-phenol | 1,3-Dihydroxybenzol |
| hellblond | | |
| **4** | 4-Amino-2-(5-methyl-thiophen-2-yl)-phenol-Hydrochlorid | 1,3-Dihydroxybenzol |
| hellblond | | |
| **5** | 4-Amino-2-(5-nitro-thiophen-2-yl)-phenol Hydrochlorid | 1,3-Dihydroxybenzol |
| hellblond | | |
| **6** | 4-Amino-2-furan-2-yl-phenol-Hydrochlorid | 1,3-Dihydroxybenzol |
| hellblond | | |
| **7** | 4-Amino-2-(2-thienyl)-phenol | 1,3-Diamino-4-(2'-hydroxyethoxy)-benzol-sulfat |
| rotviolett | | |
| **8** | 4-Amino-2-(5-methyl-thiophen-2-yl)-phenol-Hydrochlorid | 1,3-Diamino-4-(2'-hydroxyethoxy)-benzol-sulfat |
| rotviolett | | |
| **9** | 4-Amino-2-(5-nitro-thiophen-2-yl)-phenol Hydrochlorid | 1,3-Diamino-4-(2'-hydroxyethoxy)-benzol-sulfat |
| braun | | |
| **10** | 4-Amino-2-furan-2-yl-phenol-Hydrochlorid | 1,3-Diamino-4-(2'-hydroxyethoxy)-benzol-sulfat |
| braun | | |
| **11** | 4-Amino-2-(2-thienyl)-phenol | 5-Amino-2-methyl-phenol |
| rotorange | | |
| **12** | 4-Amino-2-(5-methyl-thiophen-2-yl)-phenol-Hydrochlorid | 5-Amino-2-methyl-phenol |
| rotorange | | |
| **13** | 4-Amino-2-(5-nitro-thiophen-2-yl)-phenol Hydrochlorid | 5-Amino-2-methyl-phenol |
| braun | | |
| **14** | 4-Amino-2-furan-2-yl-phenol-Hydrochlorid | 5-Amino-2-methyl-phenol |
| braun | | |
| **15** | 4-Amino-2-(2-thienyl)-phenol | 1-Naphthol |
| violett | | |
| **16** | 4-Amino-2-(5-methyl-thiophen-2-yl)-phenol-Hydrochlorid | 1-Naphthol |
| violett | | |
| **17** | 4-Amino-2-(5-nitro-thiophen-2-yl)-phenol Hydrochlorid | 1-Naphthol |
| kupferfarben | | |
| **18** | 4-Amino-2-furan-2-yl-phenol-Hydrochlorid | 1-Naphthol |
| kupferfarben | | |

Alle in der vorliegenden Anmeldung enthaltenen Prozentangaben stellen soweit nicht anders angegeben Gewichtsprozente dar.

## Patentansprüche

1. p-Aminophenol-Derivat der allgemeinen Formel (I) oder dessen physiologisch verträgliches, wasserlösliches Salz, 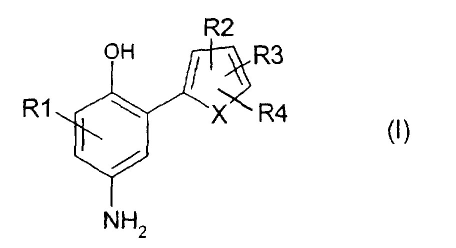 worin
**X** gleich Sauerstoff, Schwefel, oder NR5 ist;
**R1** gleich Wasserstoff, einem Halogenatom, einer C₁-C₄-Alkylgruppe, einer C₁-C₄-Hydroxyalkylgruppe oder einer C₁-C₄-Alkoxygruppe ist;
**R2** und **R4** unabhänging voneinander gleich Wasserstoff, einer Hydroxygruppe, einem Halogenatom, einer Cyanogruppe, einer C₁-C₄-Alkoxygruppe, einer C₁-C₆-Alkylgruppe, einer C₁-C₄-Alkylthioethergruppe, einer Mercaptogruppe, einer Nitrogruppe, einer Aminogruppe, einer C₁-C₆ -Alkylaminogruppe, einer (C₁-C₆)-Dialkylaminogruppe, einer -C(O)H-Gruppe, einer -C(O)CH₃-Gruppe, einer -C(O)CF₃-Gruppe, einer -Si(CH₃)₃-Gruppe, einer C₁-C₄-Hydroxyalkylgruppe, einer C₃-C₄-Dihydroxyalkylgruppe, einer -CH=CHR6-Gruppe, einer -(CH₂)ₚ-CO₂R7-Gruppe oder einer -(CH₂)ₚ-R8-Gruppe (mit p=1,2,3 oder 4), einer -C(R9)=NR10-Gruppe oder einer C(R11)H-NR12R13-Gruppe sind;
**R3** gleich Wasserstoff, einem Halogenatom, einer C₁-C₆ Alkylgruppe oder einer -C(O)H-Gruppe ist;
**R5** gleich Wasserstoff, einer C₁-C₆-Alkylgruppe, einer C₁-C₄-Hydroxyalkylgruppe, einer Phenylgruppe oder einer Acetylgruppe ist;
**R6** gleich Wasserstoff, einer Hydroxygruppe, einer Nitrogruppe, einer Aminogruppe, einer -CO₂R7-Gruppe oder einer -C(O)CH₃-Gruppe ist;
**R7, R9 und R11** unabhänging voneinander gleich Wasserstoff oder einer C₁-C₄-Alkylgruppe sind;
**R8** gleich einer Aminogruppe oder einer Nitrilgruppe ist;
**R10, R12 und R13** unabhängig voneinander gleich Wasserstoff, einer Hydroxygruppe, einer C₁-C₄-Alkylgruppe, einer C₁-C₄-Hydroxyalkylgruppe, einer C₃-C₄-Dihydroxyalkylgruppe oder einem Rest der Formel (II) sind 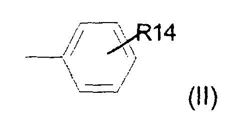 und
**R14** gleich Wasserstoff, einer Aminogruppe oder einer Hydroxygruppe ist, unter der Bedingung, dass mindestens einer der Reste **R1** bis **R4** von Wasserstoff verschieden ist, wenn **X** gleich Schwefel ist.

2. p-Aminophenol-Derivat nach Anspruch 1, **dadurch gekennzeichnet, dass** es ausgewählt ist aus der Gruppe bestehend aus 4-Amino-2-(2-furyl)-phenol; 4-Amino-2-(pyrrol-2-yl)-phenol; 4-Amino-2-(1-methyl-1Hpyrrol-2-yl)-phenol; 4-Amino-3-chlor-2-(2-thienyl)-phenol; 4-Amino-3-methyl-2-(2-thienyl)-phenol; 4-Amino-5-chlor-2-(2-thienyl)-phenol; 4-Amino-5-methyl-2-(2-thienyl)-phenol; 4-Amino-6-chlor-2-(2-thienyl)-phenol; 4-Amino-6-methyl-2-(2-thienyl)-phenol; 4-Amino-2-(3-acetyl-2-thienyl)-phenol; 4-Amino-2-(3-chlor-2-thienyl)-phenol; 4-Amino-2-(3-formyl-2-thienyl)-phenol; 4-Amino-2-(3-methyl-2-thienyl)-phenol; 4-Amino-2-(3-nitro-2-thienyl)-phenol; 4-Amino-2-(4-acetyl-2-thienyl)-phenol; 4-Amino-2-(4-chlor-2-thienyl)-phenol; 4-Amino-2-(4-formyl-2-thienyl)-phenol; 4-Amino-2-(4-methyl-2-thienyl)-phenol; 4-Amino-2-(4-nitro-2-thienyl)-phenol; 4-Amino-2-(5-acetyl-2-thienyl)-phenol; 4-Amino-2-(5-chlor-2-thienyl)-phenol; 4-Amino-2-(5-formyl-2-thienyl)-phenol; 4-Amino-2-(5-methyl-2-thienyl)-phenol; 4-Amino-2-(5-nitro-2-thienyl)-phenol sowie deren physiologisch verträglichen Salzen.

3. p-Aminophenol-Derivat nach Anspruch 1 oder 2, **dadurch gekennzeichnet, dass** in der Formel (I) (i) **R1** gleich Wasserstoff ist und/oder (ii) mindestens einer der Reste **R2 , R3** und **R4** Wasserstoff oder eine Methylgruppe bedeuten und/oder (iii) **X** gleich Schwefel oder Sauerstoff ist, unter der Bedingung, dass mindestens einer der Reste **R1** bis **R4** von Wasserstoff verschieden ist, wenn **X** gleich Schwefel ist.

4. p-Aminophenol-Derivat nach einem der Ansprüche 1 bis 3, **dadurch gekennzeichnet, dass** es ausgewählt ist aus der Gruppe bestehend aus 4-Amino-2-(3-methyl-2-thienyl)-phenol und 4-Amino-2-(5-methyl-2-thienyl)-phenol sowie deren physiologisch verträglichen Salzen.

5. Mittel zum oxidativen Färben von Keratinfasem auf der Basis einer Entwicklersubstanz-Kupplersubstanz-Kombination, **dadurch gekennzeichnet, dass** es als Entwicklersubstanz mindestens ein p-Aminophenol-Derivat der Formel (I) nach einem der Ansprüche 1 bis 4 enthält.

6. Mittel nach Anspruch 5, **dadurch gekennzeichnet, dass** es das p-Aminophenol-Derivat der Formel (I) in einer Menge von 0,005 bis 20,0 Gewichtsprozent enthält.

7. Mittel nach Anspruch 5 oder 6, **dadurch gekennzeichnet, dass** die Kupplersubstanz ausgewählt ist aus der Gruppe bestehend aus 2,6-Diamino-pyridin, 2-Amino-4-[(2-hydroxyethyl)amino]-anisol, 2,4-Diamino-1-fluor-5-methyl-benzol, 2,4-Diamino-1-methoxy-5-methylbenzol, 2,4-Diamino-1-ethoxy-5-methyl-benzol, 2,4-Diamino-1-(2-hydroxyethoxy)-5-methyl-benzol, 2,4-Di[(2-hydroxyethyl)amino]-1,5-dimethoxy-benzol, 2,3-Diamino-6-methoxy-pyridin, 3-Amino-6-methoxy-2-(methylamino)-pyridin, 2,6-Diamino-3,5-dimethoxy-pyridin, 3,5-Diamino-2,6-dimethoxy-pyridin, 1,3-Diamino-benzol, 2,4-Diamino-1-(2-hydroxyethoxy)-benzol, 2,4-Diamino-1,5-di(2-hydroxyethoxy)-benzol, 1-(2-Aminoethoxy)-2,4-diamino-benzol, 2-Amino-1-(2-hydroxyethoxy)-4-methylamino-benzol, 2,4-Diaminophenoxy-essigsäure, 3-[Di(2-hydroxyethyl)amino]-anilin, 4-Amino-2-di[(2-hydroxyethyl)amino]-1-ethoxy-benzol, 5-Methyl-2-(1-methylethyl)-phenol, 3-[(2-Hydroxyethyl)amino]-anilin, 3-[(2-Aminoethyl)-amino]-anilin, 1,3-Di(2,4-diaminophenoxy)-propan, Di(2,4-diamino-phenoxy)-methan, 1,3-Diamino-2,4-dimethoxy-benzol, 2,6-Bis(2-hydroxyethyl)amino-toluol, 4-Hydroxyindol, 3-Dimethyl-aminophenol, 3-Diethylamino-phenol, 5-Amino-2-methyl-phenol, 5-Amino-4-fluor-2-methyl-phenol, 5-Amino-4-methoxy-2-methyl-phenol, 5-Amino-4-ethoxy-2-methyl-phenol, 3-Amino-2,4-dichlor-phenol, 5-Amino-2,4-dichlorphenol, 3-Amino-2-methyl-phenol, 3-Amino-2-chlor-6-methyl-phenol, 3-Amino-phenol, 2-[(3-Hydroxyphenyl)amino]-acetamid, 5-[(2-Hydroxyethyl)amino]-2-methyl-phenol, 3-[(2-Hydroxyethyl)amino]-phenol, 3-[(2-Methoxyethyl)-amino]-phenol, 5-Amino-2-ethyl-phenol, 2-(4-Amino-2-hydroxyphenoxy)-ethanol, 5-[(3-Hydroxypropyl)amino]-2-methyl-phenol, 3-[(2,3-Dihydroxy-propyl)amino]-2-methyl-phenol, 3-[(2-Hydroxyethyl)amino]-2-methyl-phenol, 2-Amino-3-hydroxy-pyridin, 5-Amino-4-chlor-2-methyl-phenol, 1-Naphthol, 1,5-Dihydroxy-naphthalin, 1,7-Dihydroxy-naphthalin, 2,3-Dihydroxy-naphthalin, 2,7-Dihydroxy-naphthalin, 2-Methyl-1-naphthol-acetat, 1,3-Dihydroxy-benzol, 1-Chlor-2,4-dihydroxy-benzol, 2-Chlor-1,3-dihydroxy-benzol, 1,2-Dichlor-3,5-dihydroxy-4-methyl-benzol, 1,5-Dichlor-2,4-dihydroxy-benzol, 1,3-Dihydroxy-2-methyl-benzol, 3,4-Methylendioxy-phenol, 3,4-Methylendioxy-anilin, 5-[(2-Hydroxyethyl)amino]-1,3-benzodioxol, 6-Brom-1-hydroxy-3,4-methylendioxy-benzol, 3,4-Diamino-benzoesäure, 3,4-Dihydro-6-hydroxy-1,4(2H)-benzoxazin, 6-Amino-3,4-dihydro-1,4(2H)-benzoxazin, 3-Methyl-1-phenyl-5-pyrazolon, 5,6-Dihydroxy-indol, 5,6-Dihydroxy-indolin, 5-Hydroxyindol, 6-Hydroxy-indol, 7-Hydroxy-indol und 2,3-Indolindion.

8. Mittel nach einem der Ansprüche 5 bis 7, **dadurch gekennzeichnet, dass** die Entwicklersubstanzen und Kupplersubstanzen, bezogen auf die Gesamtmenge des Färbemittels, jeweils in einer Gesamtmenge von 0,005 bis 20 Gewichtsprozent enthalten sind.

9. Mittel nach einem der Ansprüche 5 bis 8 , **dadurch gekennzeichnet, dass** es zusätzlich mindestens einen direktziehenden Farbstoff enthält.

10. Verwendung eines Mittels nach einem der Ansprüche 5 bis 9 als Haarfärbemittel.

## Claims

1. p-Aminophenol derivative of the general formula (I) or physiologically compatible, water-soluble salt thereof 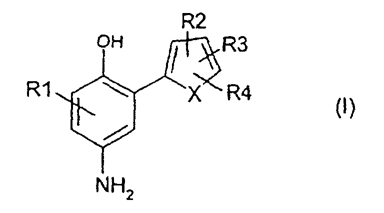 in which
**X** is oxygen, sulphur or NR5;
**R1** is hydrogen, a halogen atom, a C₁-C₄-alkyl group, a C₁-C₄-hydroxyalkyl group or a C₁-C₄-alkoxy group;
**R2** and **R4,** independently of one another, are hydrogen, a hydroxy group, a halogen group, a cyano group, a C₁-C₄-alkoxy group, a C₁-C₆-alkyl group, a C₁-C₄-alkylthioether group, a mercapto group, a nitro group, an amino group, a C₁-C₆-alkylamino group, a (C₁-C₆)-dialkylamino group, a -C(O)H group, a -C(O)CH₃ group, a -C(O)CF₃ group, a -Si(CH₃)₃ group, a C₁-C₄-hydroxyalkyl group, a C₃-C₄-dihydroxyalkyl group, a -CH=CHR6 group, a -(CH₂)ₚ-CO₂R7 group or a -(CH₂)ₚR8 group (where p = 1, 2, 3 or 4), a -C(R9)=NR10 group or a C(R11)H-NR12R13 group;
**R3** is hydrogen, a halogen atom, a C₁-C₆-alkyl group or a -C(O)H group;
**R5** is hydrogen, a C₁-C₆-alkyl group, a C₁-C₄-hydroxyalkyl group, a phenyl group or an acetyl group;
R6 is hydrogen, a hydroxy group, a nitro group, an amino group, a -CO₂R7 group or a -C(O)CH₃ group;
**R7, R9 and R11,** independently of one another, are hydrogen or a C₁-C₄-alkyl group;
**R8** is an amino group or a nitrile group;
**R10, R12 and R13,** independently of one another, are hydrogen, a hydroxy group, a C₁-C₄-alkyl group, a C₁-C₄-hydroxyalkyl group, a C₃-C₄-dihydroxyalkyl group or a radical of the formula (II) 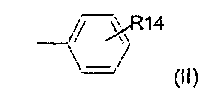 and
**R14** is hydrogen, an amino group or a hydroxy group,
with the condition that at least one of the radicals **R1** to **R4** is different from hydrogen if **X** is sulphur.

2. p-Aminophenol derivative according to Claim 1, **characterized in that** it is chosen from the group consisting of 4-amino-2-(2-furyl)phenol; 4-amino-2-(pyrrol-2-yl)phenol; 4-amino-2-(1-methyl-1H-pyrrol-2-yl)phenol; 4-amino-3-chloro-2-(2-thienyl)phenol; 4-amino-3-methyl-2-(2-thienyl)phenol; 4-amino-5-chloro-2-(2-thienyl)phenol; 4-amino-5-methyl-2-(2-thienyl)-phenol; 4-amino-6-chloro-2-(2-thienyl)phenol; 4-amino-6-methyl-2-(2-thienyl)phenol; 4-amino-2-(3-acetyl-2-thienyl)phenol; 4-amino-2-(3-chloro-2-thienyl)phenol; 4-amino-2-(3-formyl-2-thienyl)phenol; 4-amino-2-(3-methyl-2-thienyl)phenol; 4-amino-2-(3-nitro-2-thienyl)-phenol; 4-amino-2-(4-acetyl-2-thienyl)phenol; 4-amino-2-(4-chloro-2-thienyl)phenol; 4-amino-2-(4-formyl-2-thienyl)phenol; 4-amino-2-(4-methyl-2-thienyl)phenol; 4-amino-2-(4-nitro-2-thienyl)phenol; 4-amino-2-(5-acetyl-2-thienyl)phenol; 4-amino-2-(5-chloro-2-thienyl)phenol; 4-amino-2-(5-formyl-2-thienyl)phenol; 4-amino-2-(5-methyl-2-thienyl)phenol; 4-amino-2-(5-nitro-2-thienyl)phenol and physiologically compatible salts thereof.

3. p-Aminophenol derivative according to Claim 1 or 2, **characterized in that**, in the formula (I), (i) **R1** is hydrogen and/or (ii) at least one of the radicals **R2, R3** and **R4** is hydrogen or a methyl group and/or (iii) **X** is sulphur or oxygen, with the condition that at least one of the radicals **R1** to **R4** is different from hydrogen if **X** is sulphur.

4. p-Aminophenol derivative according to one of Claims 1 to 3, **characterized in that** it is chosen from the group consisting of 4-amino-2-(3-methyl-2-thienyl)phenol and 4-amino-2-(5-methyl-2-thienyl)phenol and physiologically compatible salts thereof.

5. Agent for the oxidative colouring of keratin fibres based on a developer substance-coupler substance combination, **characterized in that** it comprises at least one p-aminophenol derivative of the formula (I) according to one of Claims 1 to 4 as developer substance.

6. Agent according to Claim 5, **characterized in that** it comprises the p-aminophenol derivative of the formula (I) in an amount of from 0.005 to 20.0% by weight.

7. Agent according to Claim 5 or 6, **characterized in that** the coupler substance is chosen from the group consisting of 2,6-diaminopyridine, 2-amino-4-[(2-hydroxyethyl)amino]anisole, 2,4-diamino-1-fluoro-5-methylbenzene, 2,4-diamino-1-methoxy-5-methylbenzene, 2,4-diamino-1-ethoxy-5-methylbenzene, 2,4-diamino-1-(2-hydroxyethoxy)-5-methylbenzene, 2,4-di[(2-hydroxyethyl)amino]-2,5-dimethoxybenzene, 2,3-diamino-6-methoxypyridine, 3-amino-6-methoxy-2-(methylamino)pyridine, 2,6-diamino-3,5-dimethoxypyridine, 3,5-diamino-2,6-dimethoxypyridine, 1,3-diaminobenzene, 2,4-diamino-1-(2-hydroxyethoxy)benzene, 2,4-diamino-1,5-di(2-hydroxyethoxy)benzene, 1-(2-aminoethoxy)-2,4-diaminobenzene, 2-amino-1-(2-hydroxyethoxy)-4-methylaminobenzene, 2,4-diaminophenoxyacetic acid, 3-[di(2-hydroxyethyl)amino]-aniline, 4-amino-2-di[(2-hydroxyethyl)amino]-1-ethoxybenzene, 5-methyl-2-(1-methylethyl)phenol, 3-[(2-hydroxyethyl)amino]aniline, 3-[(2-aminoethyl)amino]-aniline, 1,3-di(2,4-diaminophenoxy)propane, di(2,4-diaminophenoxy)methane, 1,3-diamino-2,4-dimethoxybenzene, 2,6-bis(2-hydroxyethyl)aminotoluene, 4-hydroxyindole, 3-dimethylaminophenol, 3-diethylaminophenol, 5-amino-2-methylphenol, 5-amino-4-fluoro-2-methylphenol, 5-amino-4-methoxy-2-methylphenol, 5-amino-4-ethoxy-2-methylphenol, 3-amino-2,4-dichlorophenol, 5-amino-2,4-dichlorophenol, 3-amino-2-methylphenol, 3-amino-2-chloro-6-methylphenol, 3-aminophenol, 2-[(3-hydroxyphenyl)amino]acetamide, 5-[(2-hydroxyethyl)amino]-2-methylphenol, 3-[(2-hydroxyethyl)-amino]phenol, 3-[(2-methoxyethyl)amino]phenol, 5-amino-2-ethylphenol, 2-(4-amino-2-hydroxyphenoxy)ethanol, 5-[(3-hydroxypropyl)amino]-2-methylphenol, 3-[(2,3-dihydroxypropyl)amino]-2-methylphenol, 3-[(2-hydroxyethyl)amino]-2-methylphenol, 2-amino-3-hydroxypyridine, 5-amino-4-chloro-2-methylphenol, 1-naphthol, 1,5-dihydroxynaphthalene, 1,7-dihydroxynaphthalene, 2,3-dihydroxynaphthalene, 2,7-dihydroxynaphthalene, 2-methyl-1-naphthol acetate, 1,3-dihydroxybenzene, 1-chloro-2,4-dihydroxybenzene, 2-chloro-1,3-dihydroxybenzene, 1,2-dichloro-3,5-dihydroxy-4-methylbenzene, 1,5-dichloro-2,4-dihydroxybenzene, 1,3-dihydroxy-2-methylbenzene, 3,4-methylenedioxyphenol, 3,4-methylenedioxyaniline, 5-[(2-hydroxyethyl)amino]-1,3-benzodioxol, 6-bromo-1-hydroxy-3,4-methylenedioxybenzene, 3,4-diaminobenzoic acid, 3,4-dihydro-6-hydroxy-1,4(2H)-benzoxazine, 6-amino-3,4-dihydro-1,4(2H)-benzoxazine, 3-methyl-1-phenyl-5-pyrazolone, 5,6-dihydroxyindole, 5,6-dihydroxyindoline, 5-hydroxyindole, 6-hydroxyindole, 7-hydroxyindole and 2,3-indolinedione.

8. Agent according to one of Claims 5 to 7, **characterized in that** the developer substances and coupler substances, based on the total amount of the colorant, are in each case present in a total amount of from 0.005 to 20% by weight.

9. Agent according to one of Claims 5 to 8, **characterized in that** it additionally comprises at least one direct dye.

10. Use of an agent as claimed in one of Claims 5 to 9 as hair colorant.

## Revendications

1. Dérivé de p-aminophénol de formule générale (I) ou sel hydrosoluble physiologiquement acceptable d'un tel dérivé, 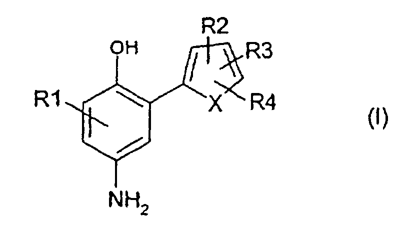 formule dans laquelle
**X** représente un atome d'oxygène ou de soufre ou NR5 ;
**R1** représente un atome d'hydrogène ou d'halogène, ou un groupe alkyle en C₁-C₄, un groupe hydroxyalkyle en C₁-C₄ ou un groupe alcoxy en C₁-C₄ ;
**R2** et **R4** représentent, indépendamment l'un de l'autre, un atome d'hydrogène ou d'halogène ou un groupe hydroxy un groupe cyano, un groupe alcoxy en C₁-C₄, un groupe alkyle en C₁-C₆, un groupe alkyl(C₁-C₄)thioéther, un groupe mercapto, un groupe nitro, un groupe amino, un groupe alkyl (C₁-C₆) amino, un groupe dialkyl (C₁-C₆) amino, un groupe -C(O)H, un groupe -C (O) CH₃, un groupe -C(O)CF₃, un groupe -Si (CH₃)₃, un groupe hydroxyalkyle en C₁-C₄, un groupe dihydroxyalkyle en C₃-C₄, un groupe -CH=CHR6, un groupe - (CH₂)ₚ-CO₂R7 ou un groupe - (CH₂)ₚ-R8 (où p = 1, 2, 3 ou 4), un groupe -C(R9)=NR10 ou un groupe C(R11)H-NR12R13 ;
**R3** représente un atome d'hydrogène ou d'halogène, ou un groupe alkyle en C₁-C₆ ou un groupe -C(O)H ;
**R5** représente un atome d'hydrogène, un groupe alkyle en C₁-C₆, un groupe hydroxyalkyle en C₁-C₄, un groupe phényle ou un groupe acétyle ;
**R6** représente un atome d'hydrogène, un groupe hydroxy, un groupe nitro, un groupe amino, un groupe -CO₂R7 ou un groupe -C(O)CH₃ ;
**R7, R9** et **R11** représentent, indépendamment les uns des autres, un atome d'hydrogène ou un groupe alkyle en C₁-C₄ ;
**R8** représente un groupe amino ou un groupe nitrile ;
**R10, R12** et **R13** représentent, indépendamment les uns des autres, un atome d'hydrogène, un groupe hydroxy, un groupe alkyle en C₁-C₄, un groupe hydroxyalkyle en C₁-C₄, un groupe dihydroxyalkyle en C₃-C₄ ou un radical de formule (II) 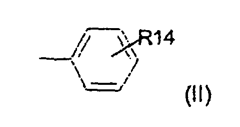 et
**R14** représente un atome d'hydrogène, un groupe amino ou un groupe hydroxy ;
étant entendu qu'au moins l'un des radicaux **R1** à **R4** est différent d'un atome d'hydrogène lorsque **X** est un atome de soufre.

2. Dérivé de p-aminophénol selon la revendication 1, **caractérisé en ce qu'**il est choisi dans le groupe constitué par le 4-amino-2-(2-furyl)phénol, le 4-amino-2-(pyrrol-2-yl)phénol, le 4-amino-2-(1-méthyl-1H-pyrrol-2-yl)phénol, le 4-amino-3-chloro-2-(2-thiényl)phénol, le 4-amino-3-méthyl-2-(2-thiényl)phénol, le 4-amino-5-chloro-2-(2-thiényl)phénol, le 4-amino-5-méthyl-2-(2-thiényl)phénol, le 4-amino-6-chloro-2-(2-thiényl)-phénol, le 4-amino-6-méthyl-2-(2-thiényl)phénol, le 4-amino-2-(3-acétyl-2-thiényl)phénol, le 4-amino-2-(3-chloro-2-thiényl)phénol, le 4-amino-2-(3-formyl-2-thiényl)phénol, le 4-amino-2-(3-méthyl-2-thiényl)phénol, le 4-amino-2-(3-nitro-2-thiényl)phénol, le 4-amino-2-(4-acétyl-2-thiényl)phénol, le 4-amino-2-(4-chloro-2-thiényl)phénol, le 4-amino-2-(4-formyl-2-thiényl)phénol, le 4-amino-2-(4-méthyl-2-thiényl)-phénol, le 4-amino-2-(4-nitro-2-thiényl)phénol, le 4-amino-2-(5-acétyl-2-thiényl)phénol, le 4-amino-2-(5-chloro-2-thiényl)phénol, le 4-amino-2-(5-formyl-2-thiényl)phénol, le 4-amino-2-(5-formyl-2-thiényl)phénol, le 4-amino-2-(5-méthyl-2-thiényl)phénol, le 4-amino-2-(5-nitro-2-thiényl)phénol ainsi que leurs sels physiologiquement acceptables.

3. Dérivé de p-aminophénol selon la revendication 1 ou 2, **caractérisé en ce que** dans la formule (I) (i) **R1** représente un atome d'hydrogène et/ou (ii) au moins l'un des radicaux **R2, R3** et **R4** représente un atome d'hydrogène ou un groupe méthyle et/ou (iii) **X** représente un atome de soufre ou d'oxygène, étant entendu qu'au moins l'un des radicaux **R1** à **R4** est différent d'un atome d'hydrogène lorsque **X** est un atome de soufre.

4. Dérivé de p-aminophénol selon l'une quelconque des revendications 1 à 3, **caractérisé en ce qu'**il est choisi dans le groupe constitué par le 4-amino-2-(3-méthyl-2-thiényl)phénol et le 4-amino-2-(5-méthyl-2-thiényl)phénol ainsi que leurs sels physiologiquement acceptables.

5. Composition pour la teinture par oxydation de fibres de kératine, à base d'une association de coupleur-développeur, **caractérisée en ce qu'**elle contient en tant que développeur au moins un dérivé de p-aminophénol de formule (I) selon l'une quelconque des revendications 1 à 4.

6. Composition selon la revendication 5, **caractérisée en ce qu'**elle contient un dérivé de p-aminophénol de formule (I) en une quantité de 0,005 à 20,0 % en poids.

7. Composition selon la revendication 5 ou 6, **caractérisée en ce que** le coupleur est choisi dans le groupe constitué par la 2,6-diaminopyridine, le 2-amino-4-[(2-hydroxyéthyl)amino]anisole, le 2,4-diamino-1-fluoro-5-méthylbenzène, le 2,4-diamino-1-méthoxy-5-méthylbenzène, le 2,4-diamino-1-éthoxy-5-méthylbenzène, le 2,4-diamino-1-(2-hydroxyéthoxy)-5-méthylbenzène, le 2,4-di[(2-hydroxyéthyl)amino]-1,5-diméthoxybenzène, la 2,3-diamino-6-méthoxypyridine, la 3-amino-6-méthoxy-2-(méthylamino)pyridine, la 2,6-diamino-3,5-diméthoxypyridine, la 3,5-diamino-2,6-diméthoxypyridine, le 1,3-diaminobenzole, le 2,4-diamino-1-(2-hydroxyéthoxy)benzène, le 2,4-diamino-1,5-di(2-hydroxyéthoxy)benzène, le 1-(2-aminoéthoxy)-2,4-diaminobenzène, le 2-amino-1-(2-hydroxyéthoxy)-4-méthylaminobenzène, l'acide 2,4-diaminophénoxyacétique, la 3-[di(2-hydroxyéthyl)amino]aniline, le 4-amino-2-di[(2-hydroxyéthyl)amino]-1-éthoxybenzène, le 5-méthyl-2-(1-méthyléthyl)phénol, la 3-[(2-hydroxyéthyl)-amino] aniline, la 3-[(2-aminoéthyl)amino]aniline, le 1,3-di(2,4-diaminophénoxy)propane, le di(2,4-diaminophénoxy)méthane, le 1,3-diamino-2,4-diméthoxybenzène, le 2,6-bis(2-hydroxyéthyl)aminotoluène, le 4-hydroxyindole, le 3-diméthylaminophénol, le 3-diéthylaminophénol, le 5-amino-2-méthylphénol, le 5-amino-4-fluoro-2-méthylphénol, le 5-amino-4-méthoxy-2-méthylphénol, le 5-amino-4-éthoxy-2-méthylphénol, le 3-amino-2,4-dichlorophénol, le 5-amino-2,4-dichlorophénol, le 3-amino-2-méthylphénol, le 3-amino-2-chloro-6-méthylphénol, le 3-aminophénol, le 2-[(3-hydroxyphényl)amino]acétamide, le 5-[(2-hydroxyéthyl)amino]-2-méthylphénol, le 3-[(2-hydroxyéthyl)amino]phénol, le 3-[(2-méthoxyéthyl)amino]phénol, le 5-amino-2-éthylphénol, le 2-(4-amino-2-hydroxyphénoxy)éthanol, le 5-[(3-hydroxypropyl)amino]-2-méthylphénol, le 3-[(2,3-dihydroxypropyl)amino]-2-méthylphénol, le 3-[(2-hydroxyéthyl)amino]-2-méthylphénol, la 2-amino-3-hydroxypyridine, le 5-amino-4-chloro-2-méthylphénol, le 1-naphtol, le 1,5-dihydroxynaphtalène, le 1,7-dihydroxynaphtalène, le 2,3-dihydroxynaphtalène, le 2,7-dihydroxynaphtalène, l'acétate de 2-méthyl-1-naphtol, le 1,3-dihydroxybenzène, le 1-chloro-2,4-dihydroxybenzène, le 2-chloro-1,3-dihydroxybenzène, le 1,2-dichloro-3,5-dihydroxy-4-méthylbenzène, le 1,5-dichloro-2,4-dihydroxybenzène, le 1,3-dihydroxy-2-méthylbenzène, le 3,4-méthylènedioxophénol, la 3,4-méthylènedioxyaniline, le 5-[(2-hydroxyéthyl)amino]-1,3-benzodioxole, le 6-bromo-1-hydroxy-3,4-méthylènedioxybenzène, l'acide 3,4-diaminobenzoïque, la 3,4-dihydro-6-hydroxy-1,4(2H)-benzoxazine, la 6-amino-3,4-dihydro-1,4(2H)-benzoxazine, la 3-méthyl-1-phényl-5-pyrazolone, le 5,6-dihydroxyindole, la 5,6-dihydroxyindoline, le 5-hydroxyindole, le 6-hydroxyindole, le 7-hydroxyindole et la 2,3-indolinedione.

8. Composition selon l'une quelconque des revendications 5 à 7, **caractérisée en ce que** les révélateurs et les coupleurs sont contenus chacun, par rapport à la quantité de la composition de teinture, en une quantité totale de 0,005 à 20 % en poids.

9. Composition selon l'une quelconque des revendications 5 à 8, **caractérisée en ce qu'**en outre elle contient au moins un colorant direct.

10. Utilisation d'une composition selon l'une quelconque des revendications 5 à 9, en tant que composition de teinture pour cheveux.
